Europäisches Patentamt

(19) European Patent Office

Office européen des brevets

(11) EP 0 819 968 A1

(12) **EUROPEAN PATENT APPLICATION**
published in accordance with Art. 158(3) EPC

(43) Date of publication:
21.01.1998 Bulletin 1998/04

(21) Application number: 97901819.9

(22) Date of filing: 03.02.1997

(51) Int. Cl.[6]: **G02C 13/00**

(86) International application number:
PCT/JP97/00256

(87) International publication number:
WO 97/29397 (14.08.1997 Gazette 1997/35)

(84) Designated Contracting States:
DE FR GB

(30) Priority: 05.02.1996 JP 18697/96

(71) Applicant:
TOMEY TECHNOLOGY CORPORATION
Nagoya-shi, Aichi 451 (JP)

(72) Inventors:
• SAKAI, Shin
Tomey Technology Corporation
11-33,
Aichi 451 (JP)

• KOZAWA, Takako
Tomey Technology Corporation
11-33
Aichi 451 (JP)

(74) Representative:
Paget, Hugh Charles Edward et al
MEWBURN ELLIS
York House
23 Kingsway
London WC2B 6HP (GB)

(54) **DISINFECTING/WASHING COMPOSITION FOR CONTACT LENSES AND METHOD FOR DISINFECTING AND WASHING CONTACT LENSES BY USING THE SAME**

(57) It is an object of the present invention to provide a disinfecting and cleaning composition for a contact lens which is capable of disinfecting and cleaning the contact lens in a short period of time, and a method of disinfecting and cleaning the contact lens, which method permits the contact lens to be simultaneously disinfected and cleaned by using the disinfecting and cleaning composition as described above.

The above object may be attained according to the present invention which provides a disinfecting and cleaning composition for a contact lens which includes 70-95w/v% of glycol and 0.1-5w/v% of hydrogen peroxide, the balance being water. The above object may also be attained according to the present invention which provides a method of disinfecting and cleaning a contact lens wherein the above-described contact lens disinfecting and cleaning composition is applied to the contact lens, so that the contact lens is disinfected and claeaned by rubbing the contact lens with the composition.

## Description

Technical Field

The present invention relates to a disinfecting and cleaning composition for a contact lens and a method of disinfecting and cleaning the contact lens using the composition.

Background Art

Generally, contact lenses are classified into non-water-absorbable lenses and water-absorbable contact lenses, or hard contact lenses and soft contact lenses. Theses contact lenses may be soiled with deposits such as protein and lipid during wearing of the contact lenses on the eyes, which deposits derive from tear fluid and lipid of the eyes. These deposits adhering to the contact lenses deteriorate the wearing comfort of the contact lenses as felt by the lens wearer, lower the eye sight of the lens wearer, and cause various troubles with the eyes such as hyperemia of the conjunctiva. In view of this, it is required to regularly clean the contact lenses so as to remove the deposits therefrom for safe and comfortable wearing of the contact lenses.

In a conventional method of cleaning the contact lenses for removing the deposits adhering thereto, the lipid deposits are removed from the contact lenses by rubbing the contact lenses with a cleaning agent which contains a surface active agent, while the protein deposits are removed by immersing the contact lenses in a cleaning agent which contains a proterolytic enzyme.

When the contact lenses are continuously worn on the eyes, bacteria tend to adhere to and proliferate on the surfaces of the contact lenses, especially the surfaces of the water-absorbable contact lenses. In view of this, it is required to disinfect the contact lenses every day for preventing the eyes of the lens wearer from being infected with the bacteria. Although the non-water-absorbable contact lenses are not necessarily required to be disinfected as frequently as the water-absorbable contact lenses it is desirable to disinfect the non-water-absorbable contact lenses by using a disinfectant so as to prevent proliferation of microorganisms during storage of the contact lenses which have been removed from the eyes.

For disinfecting the contact lenses, there have been practiced a thermal disinfecting method in which the contact lenses are heated for disinfection, and a chemical disinfecting method in which the contact lenses are disinfected by a chemical disinfectant. In the thermal disinfecting method, the contact lenses which are immersed in a physiological sodium chloride solution or in a physiological sodium chloride solution that contains a preservative are heated to a temperature higher than 80°C, so that the contact lenses are disinfected. In the chemical disinfecting method, the contact lenses are immersed in a disinfecting or storing liquid that contains a disinfectant, so that the the contact lenses are disinfected. Examples of the disinfectant used include: an oxidizing agent such as hydrogen peroxide; anionic chemical substances such as sorbic acid and benzoic acid; and cationic chemical substances such as benzalkonium chloride, chlorhexidine salt, salt of high-molecular biguanide, and $\alpha$-4-[1-tris(2-hydroxyethyl)ammonium-2-butenyl]poly[1-dimethylammonium-2-butenyl]-$\omega$-tris(2-hydroxyethyl) ammonium chloride.

The above-described thermal disinfecting method requires a device using an electric power source. However, it is inconvenient to carry such a device. Further, in the thermal disinfecting method, the contact lenses immersed in the physiological sodium chloride solution need to be heated for a predetermined time so as to completely disinfect the contact lenses. In addition, the contact lenses need to be cooled after the heating operation. Accordingly, the thermal disinfecting method inevitably requires a total treating time of as long as about one hour, which is a sum of the heating time and the cooling time. Further, the material of the contact lenses may be deteriorated by the heating operation effected in the thermal disinfecting method.

The chemical disinfecting method wherein the contact lenses are immersed in an aqueous solution containing the disinfectant also suffers from some problems described below. Namely, the disinfectant is adsorbed on and accumulated in the material of the contact lenses, particularly the material of the water-absorbable contact lenses. The disinfectant accumulated in the material of contact lenses is not completely removed therefrom even if the contact lenses which have been subjected to the disinfecting treatment are sufficiently rinsed with a physiological sodium chloride solution. If such contact lenses are worn on the eyes, the disinfectant adsorbed on the contact lenses is emitted therefrom, so as to irritate the mucous membranes of the eyes of the lens wearer and cause an allergy.

In view of the above, there are proposed some treating agents capable of cleaning, storing and disinfecting the contact lenses. The proposed treating agents contain a disinfectant whose concentration is as low as to prevent irritation of the mucous membranes of the eyes. In these treating agents, however, the sterilizing action is made considerably weak in order to reduce the irritation to the mucous membranes of the eyes. Therefore, the contact lenses need to be soaked in the treating agents for a period of time as long as more than four hours for providing a desired sterilizing effect.

To solve the above-described problems, JP-A-2-240199 proposes a disinfecting and cleaning liquid agent which includes, as a sterilizing component, alkylene glycol, lower alkanol and a surface active agent. This liquid agent permits the contact lens to be disinfected and cleaned in a relatively short period of time. However, since this liquid agent includes the lower alkanol, it influences the material of the contact lens to such an extent that the size of the contact lens is considerably changed. Further, the lower alkanol included in this liquid agent has a strong irritating odor that disgusts the user who effects the daily disinfecting treatment of the contact lenses on a daily basis using this liquid agent.

Among the above-described disinfectants, the hydrogen peroxide has been attracting attention since the hydrogen peroxide is easily decomposed and detoxified. The hydrogen peroxide is not excessively adsorbed on and accumulated in the material of the contact lenses. Further, the hydrogen peroxide remaining in the contact lens which has been treated can be easily removed by a neutralization reaction. Thus, the hydrogen peroxide is suitably used for disinfecting the contact lenses. In a method of sterilizing the contact lenses by using the hydrogen peroxide, the contact lenses are immersed for at least ten minutes in an aqueous solution containing the hydrogen peroxide generally in an amount of 3w/v%. When the contact lenses are disinfected by using the hydrogen peroxide, the contact lenses can be disinfected in a considerably reduced period of time, as compared with a case wherein the contact lenses are treated by the chemical disinfecting method which uses a disinfectant other than the hydrogen peroxide. However, it still takes at least thirty minutes to complete the disinfection of the contact lenses before they can be worn on the eyes, including the time required for the neutralizing treatment of the hydrogen peroxide to be effected after the disinfecting treatment of the contact lenses.

US Patent No. 5,260,021 proposes a method of simultaneously disinfecting and cleaning a contact lens by rubbing the contact lens with an aqueous solution of hydrogen peroxide which contains a gelling agent. Since the aqueous solution of hydrogen peroxide to be used in the proposed method does not have a high sterilizing action, it does not provide a sufficiently high sterilizing effect even if the contact lenses are rubbed with the above aqueous solution for several tens of seconds. Thus, the aqueous solution of hydrogen peroxide is not satisfactory as a contact lens disinfecting and cleaning composition capable of exhibiting an excellent disinfecting action in a relatively short period of time.

Disclosure of the invention

The present invention was developed in the light of the above-described situations. It is therefore an object of the present invention to provide a disinfecting and cleaning composition for a contact lens which is capable of disinfecting and cleaning the contact lens in a short period of time, and a method of simultaneously disinfecting and cleaning the contact lens by using this disinfecting and cleaning composition.

The inventors of the present invention have made an extensive study in an effort to attain the above object, and found that a combined use of propylene glycol and hydrogen peroxide in respective predetermined concentrations, as a sterilizing component, exhibits a synergistic disinfecting effect which cannot be obtained when only one of the glycol and hydrogen peroxide is used.

The above-indicated object of the present invention may be attained according to the invention which provides a disinfecting and cleaning composition for a contact lens, characterized by including 70-95w/v% of glycol and 0.1-5w/v% of hydrogen peroxide, the balance being water.

In the contact lens disinfecting and cleaning composition for a contact lens according to the present invention, the glycol and hydrogen peroxide exhibit a synergistically enhanced disinfecting effect owing to the combined use of the glycol and hydrogen peroxide in the respective predetermined concentrations. Thus, the present disinfecting and cleaning composition provides a sterilizing effect (disinfecting effect) higher than a sum of the sterilizing effects, which would be obtained when each of the glycol and hydrogen peroxide were used alone. Accordingly, the amount of the hydrogen peroxide to be included in the composition can be reduced since the concentration of the hydrogen peroxide required for providing substantially the same sterilizing effect can be lowered. Thus, the treatment for neutralizing (decomposing) the hydrogen peroxide, which is effected after the contact lens is disinfected and cleaned, can be effected in a simplified manner.

The above-described glycol is dihydric alcohol. Since the dihydric alcohol has a high degree of affinity to the lipid, the lipid is easily dissolved by the dihydric alcohol, to thereby assure an excellent cleaning effect with respect to the deposits on the contact lenses, in particular, lipid deposits derived from the tear fluid.

In one preferred form of the contact lens disinfecting and cleaning composition according to the present invention, the above-described glycol is propylene glycol. The propylene glycol per se has an excellent sterilizing action (disinfecting action). Accordingly, the present disinfecting and cleaning composition provides an enhanced sterilizing effect owing to the sterilizing action exhibited by the propylene glycol per se, in addition to the synergistically improved sterilizing effect owing to the combined use of the propylene glycol and the hydrogen peroxide. Further, the propylene glycol has an excellent cleaning action, to thereby provide a high cleaning effect.

In another preferred form of the present contact lens disinfecting and cleaning composition, the glycol is polyethylene glycol having an average molecular weight of 200-600. When the polyethylene glycol is used in the present disinfecting and cleaning composition, the synergistic effect of the glycol and hydrogen peroxide is increased, so as to provide substantially the same disinfecting effect as the propylene glycol.

The present contact lens disinfecting and cleaning composition may further contain, in addition to the above-described sterilizing component, a surface active agent in an amount of up to 10w/v%. Owing to the addition of the surface active agent to the present contact lens disinfecting and cleaning composition, it exhibits an excellent cleaning effect.

The contact lens disinfecting and cleaning composition may further contain a stabilizer for stabilizing the hydrogen peroxide, in an amount of 0.00005-0.1w/v%. The stabilizer advantageously inhibits or reduces spontaneous decomposition of the hydrogen peroxide which is included in the present composition as one disinfecting component, so that the stability of the hydrogen peroxide in the disinfecting and cleaning composition can be increased, to thereby increase the stability of the disinfecting action exhibited by the hydrogen peroxide. Thus, the present contact lens disinfecting and cleaning composition exhibits an enhanced sterilizing effect.

The above-indicated object of the invention may also be attained according to the present invention which provides a method of disinfecting and cleaning a contact lens characterized in that the contact lens is simultaneously disinfected and cleaned by rubbing the contact lens with a contact lens disinfecting and cleaning composition which includes 70-95w/v% of glycol, 0.1-5w/v% of hydrogen peroxide, and water.

In the method of disinfecting and cleaning the contact lens according to the present invention, the contact lens is disinfected and cleaned by using the contact lens disinfecting and cleaning composition including glycol and hydrogen peroxide in the respective concentrations, and water. Since the disinfecting and cleaning composition exhibits a high sterilizing action owing to the synergistic effect of the glycol and hydrogen peroxide, the contact lens can be effectively disinfected and cleaned in a considerably short period of time, for example, for several tens of seconds, by running the contact lens with the composition. Thus, the present method permits effective utilization of the properties of the disinfecting and cleaning composition.

In essence, the present invention is characterized by the combined use of the glycol and hydrogen peroxide in the respective predetermined concentrations so as to permit the contact lens disinfecting and cleaning composition to exhibit a synergistically improved disinfecting action. Further, the invention aims at disinfecting the contact lens in a simplified manner by utilizing the synergistic disinfecting action of the composition.

Best mode for carrying out the present invention

In the present contact lens disinfecting and cleaning composition, the glycol is used in combination with the hydrogen peroxide so as to provide the synergistic disinfecting action. The glycol used in the present disinfecting and cleaning composition is suitably selected by taking account of the following requirements. That is, the glycol should assure high safety with respect to a living body, namely, high safety with respect to the eye tissue. In addition, the glycol should be in a liquid form at ordinary temperature (0-40°C), and should have a suitable viscosity. In view of these requirements, it is preferable to employ ethylene glycol, propylene glycol, trimethylene glycol and polyethylene glycol, for instance. It is particularly preferable to employ the propylene glycol and polyethylene glycol. The polyethylene glycol is classified into various kinds having different values of the average molecular weight. In particular, the polyethylene glycol having the average molecular weight of 200-600 is preferably used. If the average molecular weight is smaller than the above lower limit, the viscosity of the polyethylene glycol is too low. In this case, the disinfecting and cleaning composition does not exhibit a sufficient cleaning and disinfecting effect when the contact lens is rubbed with the composition. On the other hand, if the average molecular weight of the polyethylene glycol exceeds the above upper limit, the polyethylene glycol may be in a solid form at the ordinary temperature, or in a liquid form with high viscosity. Thus, such polyethylene glycol is not suitable for the contact lens disinfecting and cleaning composition.

In general, the glycol is classified into a disinfecting glycol, such as the propylene glycol, which exhibits the sterilizing action such as the propylene glycol, and non-disinfecting glycol, such as the polyethylene glocol, which does not exhibit a sterilizing action such as the polyethylene glycol. Any one of the disinfecting glycol and non-disinfecting glycol may be employed. When the disinfecting glycol is used in the present contact lens disinfecting and cleaning composition, the composition exhibits enhanced disinfecting action. Even if the non-disinfecting glycol is used, it provides the synergistic disinfecting action owing to the combined use with the hydrogen peroxide, thereby assuring an excellent sterilizing effect.

The glycol is contained in the contact lens disinfecting and cleaning composition with a concentration in a range of 70-95w/v%. If the concentration of the glycol is lower than 70w/v%, the disinfecting effect provided by the combined use with the hydrogen peroxide is insufficient. In this case, it is required to use the hydrogen peroxide with a higher concentration for disinfecting the contact lenses in a relatively short period of time. This is not favorable in view of safety. On the other hand, if the concentration of the hydrogen peroxide included in the disinfecting and cleaning composition exceeds 95w/v%, the amount of water to be included in the composition is inevitably reduced. The reduction of the amount of water to be included in the disinfecting and cleaning composition undesirably causes deterioration of solubility of additional components such as a surface active agent, a buffer and a stabilizer for stabilizing the hydrogen peroxide when these additional components are added to the disinfecting and cleaning component.

The hydrogen peroxide is included in the present contact lens disinfecting and cleaning composition as the disinfecting component with a concentration of 0.1-5w/v%. If the concentration of the hydrogen peroxide is lower than 0.1w/v%, it does not exhibit a sufficient disinfecting action. The concentration of the hydrogen peroxide exceeding the upper limit of 5w/v% is not favorable in view of the safety. The concentration of the hydrogen peroxide is suitably determined depending upon the amount and kind of the above-described glycol. Described more specifically, when the amount of the glycol included in the disinfecting and cleaning composition is relatively large, or when the disinfecting glycol is included in the composition, the concentration of the hydrogen peroxide to be included in the disinfecting and cleaning composition is made relatively low. On the other hand, when the amount of the glycol included in the composition is relatively small, or when the non-disinfecting glycol is used, the concentration of the hydrogen peroxide is made relatively high. If the propylene glycol as the disinfecting glycol is included in the disinfecting and cleaning composition, for instance, the concentration of the hydrogen peroxide is made as low as 0.1-1w/v%. On the contrary, if the polyethylene glycol as the non-disinfecting glycol is included in the disinfecting and cleaning composition, it is preferable that concentration of the hydrogen peroxide be in a range of 2-5w/v%.

The contact lens disinfecting and cleaning composition according to the present invention may further contain a predetermined surface active agent for improving the effect of removing the deposits such as the lipid included in the tear fluid and adhering to the contact lenses, and for increasing the viscosity of the solution of the disinfecting and cleaning composition. Any known surface active agents may be employed, provided that they assure a high degree of safety with respect to the living body and they do not adversely influence the material of the contact lenses. It is preferable to use a nonionic surface active agent.

The nonionic surface active agent is contained in the present contact lens disinfecting and cleaning composition, generally in an amount of 0.01-10w/v%, preferably in an amount of 1-5w/v%. If the amount of the nonionic surface active agent is smaller than 0.01w/v%, the cleaning effect to be exhibited by the disinfecting and cleaning composition tens to be insufficient. On the other hand, the amount of the nonionic surface active agent exceeding the upper limit of 10w/v% does not significantly improve the cleaning effect to be obtained. Examples of such a non-ionic surface active agent are polyoxyethylene-polyoxypropylene block polymer, condensation product of polyoxyethylene and ethylenediamine, glycerin fatty acid ester, alkylester of sucrose, polyoxyethylene alkylamine, polyoxyethylene sorbitan fatty acid ester, triethanolamine fatty acid ester, polyoxyethylene alkylether, polyoxyethylene alkylphenylether, and polyoxyethylene polystyrylphenylether. In particular, the polyoxyethylene-polyoxypropylene block polymer is favorably in view of the safety.

The present contact lens disinfecting and cleaning composition has a pH preferably in a range of 1-8, more preferably in a range of 3-7. The pH outside the above range deteriorates the stability of the hydrogen peroxide, leading to an insufficient disinfecting effect.

For keeping the pH of the contact lens disinfecting and cleaning composition within the above range, a buffer is added to the composition. The buffer is suitably selected form among any known buffers such as phosphoric acid buffer, boric acid buffer, citric acid buffer, tris buffer and carbonic acid buffer. In particular, the phosphoric acid buffer is preferable for stabilizing the hydrogen peroxide to a higher extent. The buffer is included in the disinfecting and cleaning composition, generally in an amount of 0.01-2w/v%, preferably in an amount of 0.02-1w/v%. If the amount of the buffer is lower than 0.01w/v%, it is difficult to keep the pH of the disinfecting and cleaning liquid at a constant level. The amount of the buffer exceeding the above upper limit of 2w/v% does not significantly improve the stability of the pH.

The contact lens disinfecting and cleaning composition according to the present invention may further contain a stabilizer for stabilizing the hydrogen peroxide. The stabilizer advantageously prevents decomposition and disappearing of the hydrogen peroxide, so that the disinfecting effect to be exhibited by the hydrogen peroxide is effectively stabilized. As such a stabilizer for stabilizing the hydrogen peroxide, it is possible to use stannate, nitrate, polyphosphate, salf of ethylene diamine tetraacetic acid, uric acid or acetonitride, for example. It is particularly preferable to employ the stannate such as sodium stannate and/or salf of ethylene diamine tetraacetic acid since they assure the effect of stabilizing the hydrogen peroxide even in a small amount, and they are not adsorbed on the material of the contact lenses.

The amount of the stabilizer for stabilizing the hydrogen peroxide to be added to the disinfecting and cleaning composition is suitably determined depending upon its kind. When the stannate is used as the stabilizer, for instance, it is added to the disinfecting and cleaning composition, generally in an amount of 0.00005-0.01w/v%, preferably in an amount of 0.0001-0.002w/v%. If the amount of the stannate to be added is smaller than 0.00005w/v%, the hydrogen peroxide is not sufficiently stabilized. The amount of the stannate larger than 0.01w/v% is not favorable in view of the safety. When the salt of ethylene diamine tetraacetic acid is used as the stabilizer, for instance, it is added to the disinfecting and cleaning composition generally in an amount of 0.005-0.1w/v%, preferably in an amount of 0.01-0.075w/v%. The amount of the salt of ethylene diamine tetraacetic acid outside the above range does not stabilize the hydrogen peroxide to a sufficient extent.

The present contact lens disinfecting and cleaning composition is obtained by dissolving, in a mixed solution consisting of water and a predetermined concentration of glycol, a predetermined concentration of hydrogen peroxide and additional components such as a buffer, a surface active agent and a stabilizer for stabilizing the hydrogen peroxide, which are added as needed.

In the present method of cleaning and disinfecting a contact lens, the disinfecting and cleaning composition as described above is applied to the contact lens, and the contact lens is rubbed with the composition for several tens of seconds, so that the contact lens can be simultaneously cleaned and disinfected in a simplified manner in a relatively short period of time.

Described in detail, several droplets of the contact lens disinfecting and cleaning composition are applied to the contact lens to be treated. Then, the contact lens is rubbed for 20-30 seconds with the contact lens held by and between the thumb and the forefinger, or placed on the palm. Subsequently, the contact lens is rinsed with a physiologically isotonic solution, so that the disinfecting and cleaning treatment of the contact lens is completed. The physiologically isotonic solution used for rinsing the contact lens has an osmotic pressure, generally in a range of 150-400mOsm, preferably in a range of 200-350mOsm. Any known physiologically isotonic solutions may be used as long as they assure a high degree of safety with respect to the living body, and they do not adversely influence the material of the contact lens.

The contact lens can be effectively disinfected in a relatively short period of time, and at the same time cleaned for removal of the lipid deposits adhering to the contact lens, according to the series of procedure described above. Thus, the contact lens can be disinfected and cleaned in a simplified manner, eliminating the conventionally required cumbersome procedure.

Examples

To further clarify the concept of the present invention, some examples of the invention will be described. It is to be understood, however, that the present invention is not limited to the details of the illustrated examples, but may be embodied with various changes, modifications and improvements, which may occur to those skilled in the art without departing from the spirit of the present invention.

⟨Example 1⟩

There were prepared specimens Nos. 1-16 of the contact lens disinfecting and cleaning composition according to the present invention, and specimens Nos. 1-9 of the contact lens disinfecting and cleaning composition as comparative examples, so as to have the respective compositions as indicated in the following Tables 1 and 2. In these specimens, propylene glycol according to the Japanese Pharmacopoeia (available from Maruishi Pharmaceutical Co., Ltd., Japan) and polyethylene glycol 200 ("Macrogol 200" available from Nippon Oil and Fats Co., Ltd., Japan) were used as the glycol. As the hydrogen peroxide, 35w/v% of hydrogen peroxide (in conformity with the Japanese Food Additive Standard, available from MITSUBISHI GAS CHEMICAL COMPANY, Japan) was used. As the buffer, sodium dihydrogenphosphate dihydrate (available from Wako Junyaku Kogyo K.K., Japan) was used, and polyoxyethylene[196]polyoxypropylene[67]glycol ("Lutrol

# T A B L E  1

| Compositions (w/v%) | | | | | |
|---|---|---|---|---|---|
| | | propylene glycol | polyethylene glycol | hydrogen peroxide | NaH$_2$PO$_4$ ·2H$_2$O | nonionic surface active agent |
| PRESENT INVENTION | 1 | 75 | --- | 0.3 | --- | --- |
| | 2 | 75 | --- | 0.5 | --- | --- |
| | 3 | 75 | --- | 1.0 | --- | --- |
| | 4 | 80 | --- | 0.3 | --- | --- |
| | 5 | 80 | --- | 0.5 | --- | --- |
| | 6 | 80 | --- | 1.0 | --- | --- |
| | 7 | 85 | --- | 0.3 | --- | --- |
| | 8 | 85 | --- | 0.5 | --- | --- |
| | 9 | 85 | --- | 1.0 | --- | --- |
| | 10 | --- | 85 | 1.0 | --- | --- |
| | 11 | --- | 85 | 3.0 | --- | --- |
| | 12 | --- | 85 | 5.0 | --- | --- |
| | 13 | 75 | --- | 0.3 | 0.5 | 2.5 |
| | 14 | 80 | --- | 0.3 | 0.5 | 2.5 |
| | 15 | 85 | --- | 0.3 | 0.5 | 2.5 |
| | 16 | --- | 85 | 3.0 | 0.5 | 2.5 |

# T A B L E  2

| Compositions (w/v%) | | | |
|---|---|---|---|
| | | propylene glycol | polyethylene glycol | hydrogen peroxide |

| | | propylene glycol | polyethylene glycol | hydrogen peroxide |
|---|---|---|---|---|
| COMPARATIVE EXAMPLES | 1 | --- | --- | 0.3 |
| | 2 | --- | --- | 0.5 |
| | 3 | --- | --- | 1.0 |
| | 4 | --- | --- | 3.0 |
| | 5 | --- | --- | 5.0 |
| | 6 | 75 | --- | --- |
| | 7 | 80 | --- | --- |
| | 8 | 85 | --- | --- |
| | 9 | --- | 85 | --- |

F-127" available from BASF CORPORATION, U.S.A.) was used as the nonionic surface active agent.

The specimens of the contact lens disinfecting and cleaning composition prepared as described above were examined of the sterilizing effect in order to confirm the disinfecting effect to be exhibited by each of the specimens.

Initially, 28.5g of commercially available Glucose Peptone agar medium for the sterility test (available from EIKEN CHEMICAL CO., LTD., Japan) and 15g of powdered agar for bacteria medium (available from Wako Junyaku Kogyo K.K., Japan) were dissolved in 1000mL of distilled water. The thus obtained mixture was subjected to high-pressure steam sterilization at 121°C for 20 minutes, so as to provide a Glucose Peptone agar medium.

In the meantime, 40.0g of commercially available Tryptone Soya agar medium (available from EIKEN CHEMICAL CO., LTD., Japan) was dissolved in 1000mL of distilled water, and the obtained mixture was subjected to the steam-sterilization under pressure at 121°C for 20 minutes, so as to provide a Tryptone Soya agar medium.

10mL of the contact lens disinfecting and cleaning composition specimens Nos. 1-16 of the present invention and the contact lens disinfecting and cleaning composition specimens Nos. 1-9 according to the comparative examples were put into the respective test tubes. To each of the test tubes, there was added 0.05mL of bacterium or fungus liquid which contains one of the following bacteria and fungus in an amount of $10^8$cfu/mL-$10^9$cfu/mL: Candida albicans ATCC 10231, Staphylococcus aureus ATCC 6538P, Escherichia coli ATCC 8739, and Serratia marcescens ATCC 13880. Each of the obtained mixtures was agitated, to thereby provide a bacterium or fungus suspension wherein each specimen of the contact lens disinfecting and cleaning composition contains one of the bacteria and fungus in an amount of $10^6$cfu/mL-$10^7$cfu/mL. Each of the obtained suspension was kept at 23°C for 5 minutes. There was calculated a viable cell count per 1mL of each of the bacterium or fungus suspensions which were treated by the respective contact lens disinfecting and cleaning composition specimens, according to a plate dilution method by respectively using the Glucose Peptone agar medium for the suspension including the Candida albicans, and the Tryptone Soya agar medium for the suspensions including the Staphylococcus aureus, Escherichia coli and Serratia marcescens. Then, a rate of reduction of each of the bacteria or fungus was calculated in logarithm according to the following equation.

Reduction rate [in logarithm] = LOG{(the viable cell count per 1mL of each bacterium or fungus suspension immediately after the preparation) - (the viable cell count per 1mL of each bacterium or fungus suspension after the treatment by each specimen of the contact lens disinfecting and cleaning composition)}

The results are indicated in the following TABLE 3. In the plate dilution method, the samples had concentrations not inhibiting a growth of the bacteria or fungus.

As is apparent from the results indicated in the above TABLE 3, the contact lens disinfecting and cleaning composition according to the present invention which uses propylene glycol and hydrogen peroxide in combination or polyethylene glycol and hydrogen peroxide in combination exhibited an excellent disinfecting effect, whereas the contact lens disinfecting and cleaning composition according to the comparative examples which uses only one of propylene

## T A B L E 3

| | | bacteria or fungus | | | | | | bacteria of fungus | | | |
|---|---|---|---|---|---|---|---|---|---|---|---|
| | | CA | EC | SM | SA | | | CA | EC | SM | SA |
| INVENTION | 1 | 6.31 | 3.38 | 6.24 | 1.04 | COMPARATIVE EXAMPLES | 1 | 0 | 0.18 | 0.28 | 0.12 |
| | 2 | 6.31 | 4.51 | 6.24 | 1.01 | | 2 | 0 | 0.14 | 0.54 | 0.08 |
| | 3 | 6.31 | 6.61 | 6.24 | 1.87 | | 3 | 0 | 0.31 | 0.60 | 0.40 |
| | 4 | 6.31 | 6.61 | 6.24 | 1.52 | | 4 | 0.24 | 1.01 | 0.98 | 0.17 |
| | 5 | 6.31 | 6.61 | 6.24 | 1.64 | | 5 | 0.34 | 3.05 | 2.06 | 0.96 |
| | 6 | 6.31 | 6.61 | 6.24 | 2.69 | | 6 | 6.31 | 0.31 | 3.26 | 0.75 |
| | 7 | 6.31 | 6.61 | 6.24 | 3.37 | | 7 | 6.31 | 1.58 | 6.24 | 1.11 |
| | 8 | 6.31 | 6.61 | 6.24 | 4.20 | | 8 | 6.31 | 3.27 | 6.24 | 1.63 |
| | 9 | 6.31 | 6.61 | 6.24 | 4.73 | | 9 | 0.44 | 1.69 | 2.21 | 0.15 |
| PRESENT | 10 | 0.55 | 6.61 | 6.24 | 0.48 | | | | | | |
| | 11 | 0.72 | 6.61 | 6.24 | 1.79 | | | | | | |
| | 12 | 1.73 | 6.61 | 6.24 | 6.40 | | | | | | |
| | 13 | 6.31 | 6.61 | 6.24 | 1.31 | | | | | | |
| | 14 | 6.31 | 6.61 | 6.24 | 1.80 | | | | | | |
| | 15 | 6.31 | 6.61 | 6.24 | 3.20 | | | | | | |
| | 16 | 0.70 | 6.61 | 6.24 | 2.02 | | | | | | |

CA: Candida albicans

EC: Escherichia coli

SM: Serratia marcescens

SA: Staphylococcus aureus

glycol, polyethylene glycol and hydrogen peroxide did not exhibit a satisfactory disinfecting effect. In other words, the present contact lens disinfecting and cleaning composition exhibited a synergistically enhanced disinfecting effect. In general, as the amount of the hydrogen peroxide in the disinfecting and cleaning composition is increased, the synergistic disinfecting effect increases. From the comparison of the results between the specimens Nos. 13-16 of the present invention in which the buffer and the nonionic surface active agent were added and the specimens Nos. 1, 4, 7 and 11 according to the comparative examples in which neither the buffer nor the nonionic surface active agent was added, it will be understood that the buffer and the nonionic surface active agent do not adversely influence the disinfecting effect exhibited by the contact lens disinfecting and cleaning composition.

⟨Example 2⟩

Specimens Nos. 17-24 of the contact lens disinfecting and cleaning composition according to the present invention were prepared in the following manner. Initially, any one of the two stabilizers for stabilizing hydrogen peroxide as shown in the following TABLE 4 was dissolved in purified water in different amounts as also indicated in the TABLE 4. To this mixture, 0.5g of sodium dihydrogenphosphate dihydrate (available from Wako Junyaku Kogyo K.K., Japan) as the buffer was dissolved. To the thus obtained solution, there were dissolved 75g of propylene glycol according to the Japanese Pharmacopoeia (available form Maruishi Pharmaceutical Co., Ltd., Japan) and 2.5g of polyoxyethylene-polyoxypropylene block polymer ("Lutrol F-127" available from BASF CORPORATION, U.S.A.) as the nonionic surface active agent. After 0.78mL of 35w/v% hydrogen peroxide (in conformity with the Japanese Food Additive Standard, available from MITSUBISHI GAS CHEMICAL COMPANY, LTD., Japan) was added to the solution, purified water was added thereto so that the solution had a total amount of 100mL. Thus, the specimens of the contact lens disinfecting and cleaning solution were obtained. In the thus obtained contact lens disinfecting and cleaning specimens Nos. 17-24, the concentration of the hydrogen peroxide was 0.3w/v%. As the stabilizers for stabilizing the hydrogen peroxide, sodium stannate trihydrate (available from Nacalai Tesque, Inc., Japan) and disodium EDTA (ethylene diamine tetraacetic acid) dihydrate (available from Wako Junyaku Kogyo K.K., Japan) were used.

After each of the contact lens disinfecting and cleaning specimens prepared as described above was stored at a temperature of 60°C for 10 days, there was measured an amount of the hydrogen peroxide remaining in each specimen. The results are also shown in the TABLE 4.

It will be clear from the results as indicated in the above TABLE 4 that the hydrogen peroxide was advantageously stabilized in the contact lens disinfecting and cleaning composition specimens Nos. 17-23 according to the present invention each of which contains the hydrogen peroxide stabilizer. Namely, substantially 100% of the hydrogen peroxide remained in these specimens. In contrast, the amount of the hydrogen peroxide remaining in the disinfecting and cleaning composition was reduced due to the decomposition in the contact lens disinfecting and cleaning

## T A B L E   4

| | | Stabilizers for stabilizing the hydrogen peroxide (g) | | Remaining hydrogen peroxide percent (%) |
|---|---|---|---|---|
| | | $EDTA \cdot 2Na \cdot 2H_2O$ | $Na_2SnO_3 \cdot 3H_2O$ | |
| PRESENT INVENTION | 17 | 0.015 | ----- | 95 |
| | 18 | 0.03 | ----- | 97 |
| | 19 | 0.05 | ----- | 97 |
| | 20 | ----- | 0.0001 | 100 |
| | 21 | ----- | 0.0002 | 100 |
| | 22 | ----- | 0.0005 | 100 |
| | 23 | ----- | 0.001 | 100 |
| | 24 | ----- | ----- | 60 |

specimen No. 24 in which the hydrogen peroxide stabilizer was not added.

⟨Example 3⟩

In the test for checking the disinfecting effect of the above Example 2, the contact lens disinfecting and cleaning composition was examined of its disinfecting effect on the basis of the inhibitory effect on growth of the microorganism, by using the suspension consisting of the contact lens disinfecting and cleaning composition and the microorganism. In this Example 3, a test for checking the disinfecting effect of the contact lens disinfecting and cleaning composition was conducted by using contact lenses.

Initially, there were prepared fourteen soft contact lenses ("MENICON SOFT 72" available from Menicon Co., Ltd., Japan). Further, fourteen holders for holding the respective contact lenses, and fourteen containers for accommodating the respective holders and a rinsing liquid therein were prepared.

There was prepared a suspension which includes cysts of Acanthamoeba castellani ATCC 30868 in an amount of $10^6$cells/mL-$10^7$cells/mL. As well known, the Acanthamoeba castellani causes inflammation in the cornea of the human eye if the cornea is infected with the Acanthamoeba castellani. The opposite surfaces of each contact lens were uniformly coated with 0.02mL of the thus prepared suspension, i.e., 0.01mL for each surface. Each contact lens coated with the suspension was kept at room temperature for ten minutes, so that the cysts of Acanthamoeba castellani were adsorbed on the surfaces of each contact lens. To one of the thus prepared contact lenses, ten droplets of the contact lens disinfecting and cleaning composition specimen No. 17 according to the present invention prepared in the above Example 2 were applied thereto. Then, the contact lens was disinfected and cleaned by finger rubbing for 30 seconds with the contact lens placed on the palm. One more contact lens was disinfected and cleaned by using the contact lens disinfecting and cleaning composition specimen No. 17 in the same manner. Two contact lenses were disinfected and cleaned in the same manner by using each of the specimens Nos. 18-23 of the contact lens disinfecting and cleaning composition according to the present invention prepared in the above Example 2. After each of the contact lenses was disinfected and cleaned, it was sufficiently rinsed with a physiological sodium chloride solution. Then, each contact lens was supported by the holder. Subsequently, the holder was accommodated in the container filled with a 6mL of physiological sodium chloride as an immersing liquid, so that the contact lens was immersed in the immersing liquid. Five minutes later, the contact lens was taken out of the holder, and cultured at 28°C for one month by using a PYG liquid medium. The entire amount of the immersing liquid was filtered through a 0.22μm membrane filter made of cellulose acetate. Subsequently, the membrane filter was cultured at 28°C for one month by using the PYG liquid medium. After the culture, the contact lens and the immersing liquid were respectively observed for any proliferation of the Acanthamoeba castellani by using an inverted microscope. The contact lens and the immersing liquid were evaluated as indicated in the following TABLE 5 wherein "o" indicates that no proliferation of the Acanthamoeba castellani was observed while "x" indicates that proliferation of the Acanthamoeba castellani was observed.

As is apparent from the above results, since the present contact lens disinfecting and cleaning composition is capable of exhibiting an excellent disinfecting effect, no proliferation of the Acanthamoeba castellani was observed

## T A B L E 5

| Cultered substances | | Present invention | | | | | | |
|---|---|---|---|---|---|---|---|---|
| | | 17 | 18 | 19 | 20 | 21 | 22 | 23 |
| | contact lens | o | o | o | o | o | o | o |
| | immersing liquid | o | o | o | o | o | o | o |

in the contact lens and the immersing liquid treated by the present contact lens disinfecting and cleaning composition.

〈Example 4〉

In this Example 4, the contact lens disinfecting and cleaning composition is examined of its influence on the size of the contact lens. Initially, 0.0005g of sodium stannate trihydrate (available from Nacalai Tesque, Inc., Japan) as the the hydrogen peroxide stabilizer was dissolved in purified water. In this mixture, 0.5g of sodium dihydrogenphosphate dihydrate (available from Wako Junyaku Kogyo K.K., Japan) as the buffer was dissolved. To the obtained solution, 85g of propylene glycol according to the Japanese Pharmacopoeia (available from Maruishi Pharmaceutical Co., Ltd., Japan) and 3.0g of polyoxyethylene-polyoxypropylene block polymer ("Lutrol F-127" available from BASF CORPORATION, U.S.A.) as the nonionic surface active agent were added, and dissolved by heating. After 0.78mL of 35w/v% hydrogen peroxide (in conformity with the Japanese Food Additive Standard, available from MITSUBISHI GAS CHEMICAL COMPANY, INC., Japan) was added to the solution, purified water was added thereto so that the solution had a total amount of 100mL, to thereby provide a specimen No. 25 of the contact lens disinfecting and cleaning composition according to the present invention. A specimen No. 10 of the contact lens disinfecting and cleaning composition according to a comparative example was obtained in the same manner as described above, except that 85g of ethanol was used in place of 85g of propylene glycol. The concentration of the hydrogen peroxide was 0.3w/v% in the specimen No. 25 according to the present invention and the specimen No. 10 according to the comparative example. As a specimen No. 11 according to a comparative example, there was prepared a Quick Care Starting Solution" (available from Ciba Vision Corporation, Canada) which is a commercially available disinfecting and cleaning agent for a contact lens. This Quick Care Starting Solution aims at disinfecting and cleaning the contact lens in a short period of time, and contains isopropyl alcohol, alkylene glycol, water, surface active agent, for instance.

Three soft contact lenses ("MENICON SOFT 72" available from Menicon Co., Ltd., Japan) were prepared. The diameter of each contact lens was measured in a physiological sodium chloride solution kept at 20°C. Each of the three contact lenses was immersed in 3mL of each of the three specimens of the contact lens disinfecting and cleaning composition, respectively, i.e., specimen No. 25 according to the present invention and specimens Nos. 10 and 11 according to the comparative examples. The contact lenses were immersed in the respective specimens for 6 hours at room temperature. After each of the contact lenses was sufficiently rinsed with the physiological sodium chloride solution, the contact lens was immersed in 10mL of the physiological sodium chloride solution for more than 5 minutes. Thereafter, the diameter of each contact lens was measured in the physiological sodium chloride solution kept at 20°C. The change in the diameter of each contact lens was calculated on the basis of the measured value and the value measured before the contact lens was immersed in the cleaning and disinfecting liquid. The results are shown in the following TABLE 6.

## T A B L E   6

| | | Cleaning and disinfecting compositions | | |
|---|---|---|---|---|
| | | Present invention | Comparative examples | |
| | | 25 | 10 | 11 |
| diameter of the contact lens | before immersion (mm) | 13.70 | 13.62 | 13.70 |
| | after immersion (mm) | 13.81 | 14.55 | 14.35 |
| | change in the diameter value (mm) | + 0.11 | + 0.93 | + 0.65 |

It will be apparent from the results indicated in the above TABLE 6 that the diameter value of the contact lens did not substantially change even under a severe condition of 6-hour immersion in the contact lens disinfecting and cleaning specimen No. 25 according to the present invention. In contrast, the change in the diameter values of the contact lenses was relatively large when the contact lenses were immersed in the specimens Nos. 10 and 11 of the contact lens disinfecting and cleaning composition according to the comparative examples. Described more specifically, when the contact lens was immersed in the specimen No. 10 according to the comparative example wherein the ethanol was used in place of the propylene glycol which was included in the specimen No. 25 according to the present invention, the contact lens was swollen. Further, the commercially available disinfecting and cleaning composition, i.e., the specimen No. 11 according to the comparative example, also had a great influence on the size of the contact lens. thus, the specimens Nos. 10 and 11 have a problem in practical use. Accordingly, it will be understood that the present contact lens disinfecting and cleaning composition is excellent in that it does not influence the size of the contact lens.

〈Example 5〉

There was prepared an artificial lipid stain composition as specified in ISO/TC 172/SC 7/WG 5 N 35. This composition includes 0.6g of Arlacel 80, 1.6g of castor oil, 3.5g of lanolin, 0.5g of oleic acid, 0.4g of Span 85, 0.2g of cetyl alcohol, 0.2g of cholesterol, and 3.0g of cholesterol acetate. These reagents are all available from Wako Junyaku Kogyo K.K., Japan.

Next, two soft contact lenses ("MENICON SOFT 72" available from Menicon Co., Ltd., Japan) were prepared. Each of the two soft contact lenses was uniformly coated with 0.1g of the artificial lipid stain composition prepared as described above.

0.5mL of the specimen No. 25 of the contact lens disinfecting and cleaning composition according to the present invention was dropped on one of the contact lenses which was placed on a palm. The contact lens was subjected to the cleaning and disinfecting treatment for 30 seconds by finger rubbing. Thereafter, the contact lens was rinsed with a liquid which comprises 0.5w/v% of sodium chloride, 0.5w/v% of boric acid and 0.03w/v% of borax and which has an osmotic pressure of 250mmol/kg. After the contact lens was rinsed with the liquid, the appearance of the contact lens was observed. It was confirmed that the artificial lipid stain adhering to the contact lens was completely removed.

The other one of the contact lenses was treated in the same manner as described above, except that a physiological salt solution was used in place of the contact lens disinfecting and cleaning composition specimen No. 25 according to the present invention. The appearance of the contact lens was observed, and it was found that the artificial lipid stain adhering to the contact lens was not completely removed and remained on its surfaces.

It will be apparent from the results that the present contact lens disinfecting and cleaning composition is capable of exhibiting an excellent cleaning action as well as an excellent disinfecting action.

As is apparent from the above description, the contact lens disinfecting and cleaning composition according to the present invention which includes the glycol and hydrogen peroxide in the respective predetermined concentrations is capable of exhibiting a significantly enhanced disinfecting effect owing to the synergistic effects provided by the glycol and hydrogen peroxide, as compared with the conventional contact lens disinfecting and cleaning composition. Therefore, the present arrangement considerably reduces the amount of the hydrogen peroxide included in the composition, which hydrogen peroxide needs to be subjected to the neutralizing treatment after the contact lens was disinfected and cleaned. Thus, the reduction of the amount of the hydrogen peroxide leads to a reduced time required for effecting the treatment following the disinfecting and cleaning treatment of the contact lens. Accordingly, the present arrangement permits considerable reduction of the time reduced for disinfecting and cleaning the contact lens required before the contact lens can be worn on the eye.

In the method of disinfecting and cleaning the contact lens according to the present invention, the contact lens is rubbed with the disinfecting and cleaning composition as described above, so that the contact lens can be disinfected and cleaned in a relatively short period of time in a simplified manner, as compared with the conventional method of disinfecting and cleaning the contact lens.

Industrial Applicability

It will be understood from the above description that the present invention advantageously provides the contact lens disinfecting and cleaning composition which permits the disinfection and cleaning of the contact lens in a short period of time. The present invention also provides the method of disinfecting and cleaning the contact lens which permits the contact lens to be simultaneously disinfected and cleaned by using the contact lens disinfecting and cleaning composition as described above.

Claims

1. A disinfecting and cleaning composition for a contact lens, characterized by including 70-95w/v% of glycol and 0.1-5w/v% of hydrogen peroxide, the balance being water.

2. A disinfecting and cleaning composition for a contact lens according to claim 1, wherein said glycol is propylene glycol.

3. A disinfecting and cleaning composition for a contact lens according to claim 1, wherein said glycol is polyethylene glycol having an average molecular weight of 200-600.

4. A disinfecting and cleaning composition for a contact lens according to any one of claims 1-3, further including a surface active agent in an amount of up to 0.01-10w/v%.

5. A disinfecting and cleaning composition for a contact lens according to any one of claims 1-4, further including a stabilizer for stabilizing said hydrogen peroxide, in an amount of 0.00005-0.1w/v%.

6. A method of disinfecting and cleaning a contact lens characterized in that said contact lens is simultaneously disinfected and cleaned by rubbing said contact lens with a disinfecting and cleaning composition which includes 70-95w/v% of glycol, 0.1-5w/v% of hydrogen peroxide, and water.

EP 0 819 968 A1

## INTERNATIONAL SEARCH REPORT

International application No.

PCT/JP97/00256

A. CLASSIFICATION OF SUBJECT MATTER

Int. Cl$^6$   G02C13/00

According to International Patent Classification (IPC) or to both national classification and IPC

B. FIELDS SEARCHED

Minimum documentation searched (classification system followed by classification symbols)

Int. Cl$^6$   G02C13/00

Documentation searched other than minimum documentation to the extent that such documents are included in the fields searched

| | |
|---|---|
| Jitsuyo Shinan Koho | 1926 – 1996 |
| Kokai Jitsuyo Shinan Koho | 1971 – 1996 |
| Toroku Jitsuyo Shinan Koho | 1994 – 1996 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP, 07-068145, A (Allergan, Inc.), July 4, 1995 (04. 07. 95) & DK, 414286, A0 & FI, 863581, A0 & NO, 863575, A0 & PT, 83315, A & IL, 79812, A0 & NO, 863575, A & DK, 414286, A & FI, 863581, A & AU, 6166486, A1 & JP, 62-059918, A2 & EP, 219220, A1 & ZA, 8606272, A & US, 4670178, A & CN, 86106181, A & HU, 44443, A2 & US, 32672, E & CA, 1238469, A1 & PT, 83315, B & EP, 219220, B1 & AT, 42206, E & AU, 588049, B2 & NZ, 217330, A & CA, 1265079, A1 & AU, 3902089, A1 & CN, 1006845, B & NO, 163843, B & PH, 24472, A & HU, 200945, B & KR, 9008008, B1 & DK, 160742, B & AU, 609629, B2 & FI, 84409, B & SG, 59892, A & IE, 59476, B & HK, 23994, A | 1 – 6 |

[X] Further documents are listed in the continuation of Box C.    [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 28, 1997 (28. 03. 97) | April 8, 1997 (08. 04. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)

15

**INTERNATIONAL SEARCH REPORT**

International application No.

PCT/JP97/00256

| C (Continuation).   DOCUMENTS CONSIDERED TO BE RELEVANT | | |
|---|---|---|
| Category* | Citation of document, with indication, where appropriate, of the relevant passages | Relevant to claim No. |
| A | JP, 08-509136, A (Allergan, Inc.),<br>October 1, 1996 (01. 10. 96)<br>& WO, 9416743, A1 & AU, 6127994, A1 | 1 - 6 |

Form PCT/ISA/210 (continuation of second sheet) (July 1992)